# EUROPÄISCHE PATENTANMELDUNG

(11) **EP 2 135 632 A1**
(43) Veröffentlichungstag der Anmeldung: **23.12.2009**
(21) Anmeldenummer: 08011228.7
(22) Anmeldetag: 20.06.2008
(51) Int. Cl.: A61M 15/00, B05B 11/00, A61M 11/06

(54) **Inhalator**

(71) Anmelder: BOEHRINGER INGELHEIM INTERNATIONAL GMBH, 55216 Ingelheim (DE)
(72) Erfinder: Spallek, Michael, 55216 Ingelheim am Rhein (DE); Wachtel, Herbert, 55216 Ingelheim am Rhein (DE); Bickmann, Deborah, 55430 Urbar (DE)
(74) Vertreter: Gesthuysen, von Rohr & Eggert

(57) **Zusammenfassung**

Es wird ein Inhalator (1) zur treibgasfreien Zerstäubung einer Arzneimittelzubereitung vorgeschlagen. Der Inhalator erzeugt ein Aerosol mit geringer Geschwindigkeit. Der Inhalator wird mit einer Zusatzeinrichtung (23) zur Zwischenspeicherung des erzeugten Aerosols kombiniert um eine einfachere Inhalation insbesondere für Kinder zu ermöglichen.

## Beschreibung

Die vorliegende Erfindung betrifft einen Inhalator gemäß dem Oberbegriff des Anspruchs 1 sowie eine Verwendung einer Zusatzeinrichtung mit einer Kammer zur Zwischenspeicherung einer zerstäubten Arzneimittelzubereitung.

Die vorliegende Erfindung betrifft insbesondere einen sogenannten Soft-Mist-Inhalator (SMI), also Inhalatoren, die einen sich nur verhältnismäßig langsam ausbreitenden Sprühnebel (Aerosol) erzeugen. Derartige Inhalatoren im Sinne der vorliegenden Erfindung sind insbesondere Inhalatoren, bei denen ein Aerosol mit einer Geschwindigkeit von weniger als 2 m/s, bevorzugt weniger als 1,5 m/s und ganz besonders bevorzugt weniger als 1 m/s, (jeweils gemessen in einem Abstand von 10 cm von einer Austragsdüse) ausgegeben wird.

Ausgangspunkt der vorliegenden Erfindung ist ein Inhalator, wie im Grundprinzip in der WO 91/14468 A1 und in konkreter Ausgestaltung in der WO 97/12687 A1 (Fig. 6a, 6b) dargestellt. Der bekannte Inhalator weist als Reservoir für eine zu zerstäubende Arzneimittelzubereitung einen einsetzbaren, starren Behälter mit einem Innenbeutel mit der Arzneimittelzubereitung und einen Druckerzeuger mit einer Antriebsfeder zur Förderung und Zerstäubung der Arzneimittelzubereitung auf. Die Zerstäubung erfolgt treibgas frei, nämlich durch die Kraft der Antriebsfeder. Dieser Inhalator stellt einen SMI im Sinne der vorliegenden Erfindung dar.

Problematisch ist bei Inhalatoren und auch SMIs generell, daß das Auslösen der Zerstäubung der Arzneimittelzubereitung und das Einatmen koordiniert werden müssen. Dies kann für den einzelnen Benutzer schwierig sein. Insbesondere hat sich herausgestellt, daß eine derartige Koordination gerade für Kinder sehr schwierig ist. Studien haben gezeigt, daß das erzeugte Aerosol von ungeschickten Benutzern oder beispielsweise Kindern oftmals nicht optimal inhaliert wird.

Die WO 2004/091704 A1 offenbart eine Zusatzeinrichtung zur Zwischenspeicherung einer zerstäubten Arzneimittelzubereitung in einer Kammer. Die Zusatzeinrichtung wird bei einem sogenannten Metered Dose Inhaler (MDI) eingesetzt. Der MDI weist einen unter Druck stehenden Behälter auf, der die gesetzt. Der MDI weist einen unter Druck stehenden Behälter auf, der die zu zerstäubende Arzneimittelzubereitung sowie Treibgas enthält. Bei Betätigung wird die Arzneimittelzubereitung aufgrund des Treibgases mit verhältnismäßig hohem Druck und dementsprechend hoher Geschwindigkeit und mit einem hobeln Massenstrom ausgegeben, Folglich erfolgt die Ausgabe nur sehr kurzzeitig, insbesondere für weniger als 0,4 s, meistens für etwa 0,15 - 0,36 s. Die kurze Ausgabedauer ist für eine Inhalation nachteilig, da das Einatmen zur Inhalation üblicherweise wesentlich länger dauert. Die verhältnismäßig große Geschwindigkeit, von mehr als 2 m/s, oft sogar bis über 8 m/s, mit der das Aerosol üblicherweise von einem MDI abgegeben wird, ist für eine Aufnahme in die Lunge ebenfalls nachteilig, da die Partikel (Tröpfchen) des Aerosols aufgrund der hohen Geschwindigkeit bei direkter Inhalation größtenteils an der Rachenwand des Benutzers abgeschieden werden.

Die bekannte Zusatzeinrichtung ist für einen MDI vorgesehen und dient einer Verlangsamung des Aerosols, insbesondere durch Verlängerung des Strömungswegs. Aus diesem Grunde werden derartige Zusatzeinrichtungen auch Spacer genannt. Des weiteren dient die Zusatzeinrichtung einer Zwischenspeicherung des erzeugten Aerosols, damit der Benutzer genügend Zeit hat, um das Aerosol zu inhalieren.

Der vorliegenden Erfindung liegt die Aufgabe zugrunde, einen Inhalator, besonders bevorzugt einen SMI, und die Verwendung einer Zusatzeinrichtung mit einer Kammer zur Zwischenspeicherung einer zerstäubten Arzneimittelzubereitung anzugeben, wobei eine Vereinfachung der Inhalation auch von mit geringer Geschwindigkeit ausgegebenen Aerosolen ermöglicht wird und/oder wobei Probleme bei der Koordination von Einatmen und Auslösen eines Inhalators vermieden oder zumindest minimiert werden.

Die obige Aufgabe wird durch einen Inhalator gemäß Anspruch 1 oder eine Verwendung gemäß Anspruch 12 gelöst. Vorteilhafte Weiterbildungen sind Gegenstand der Unteransprüche.

Der vorliegenden Erfindung liegt die Idee zugrunde, einen tragbaren Zerstäuber zur treibgasfreien Zerstäubung einer Arzneimittelzubereitung bzw. einen SMI mit einer Zusatzeinrichtung mit einer Kammer zur Zwischenspeicherung des erzeugten Aerosols zu kombinieren, wobei die Kammer stromab einer Austragsdüse des Inhalators angeordnet wird.

Es hat sich gezeigt, daß durch die Zusatzeinrichtung auch bei einem Inhalator, der das zu inhalierende Aerosol über eine verhältnismäßig lange Zeit, vorzugsweise von mehr als 1 s, und/oder mit verhältnismäßig geringer Geschwindigkeit, vorzugsweise von weniger als 2 m/s, besonders bevorzugt weniger als 1,5 m/s, (gemessen in 10 cm Abstand von einer Austragsdüse), erzeugt, eine überraschend verbesserte Wirkstoffinhalation insbesondere bei kleinen Kindern oder bei sonstigen Personen, die Koordinationsschwierigkeiten haben, erreicht werden kann. Die Koordination des Auslösens des Inhalators, also der Aerosolerzeugung, und des Einatmens wird wesentlich vereinfacht. Das Aerosol wird vom Inhalator erzeugt und in die Kammer der Zusatzeinrichtung gesprüht. Der Benutzer kann dann durch möglichst tiefes Einatmen, das jedoch frei von Koordinations- bzw. Synchrozuisationszwängen ist, das Aerosol inhalieren.

Die vorschlagsgemäße Lösung gestattet eine besser definierte Wirkstoffinhalation mit einem letztendlich im Mittel höheren und/oder geringer schwankenden Wirkstoffanteil, der in der Lunge abgeschieden wird. Dies gestattet eine verbesserte Therapie von Kindern und/oder eine Erweiterung der Indikation bzw. den Einsatz von anderen Arzneimittelzubereitungen, Letztendlich wird dadurch auch ein treibgasfreier Inhalator bzw. SMI noch universeller einsetzbar,

Vorzugsweise weist die Zusatzeinrichtung ein Ventil auf, so daß ein Ausatmen in den Inhalator bzw. die Klammer - also ein Rückströmen von Luft von der Abgabeseite der Zusatzeinrichtung in die Kammer - wirksam verhindert werden kann.

Weitere Vorteile, Merkmale, Eigenschaften und Aspekte der vorliegenden Erfindung ergeben sich aus den Ansprüchen und der folgenden Beschreibung einer bevorzugten Ausfühnmgsform anhand der Zeichnung. Es zeigt:
- Fig. 1: einen schematischen Schnitt eines Inhalators im ungespannten Zustand;
- Fig, 2: einen schematischen, um 90° gegenüber Fig. 1 gedrehten Schnitt des Inhalators im gespannten Zustand;
- Fig. 3: einen schematischen, zu Fig. 1 korrespondierenden Schnitt des Inhalators mit angeschlossener Zusatzeinrichtung;
- Fig, 4: eine schematische, explosionsartige Darstellung des Zerstäubers und der Zusatzeinrichtung mit verschiedenen Anbauteilen; und
- Fig. 5: eine schematische Darstellung von Versuchsergebnissen.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

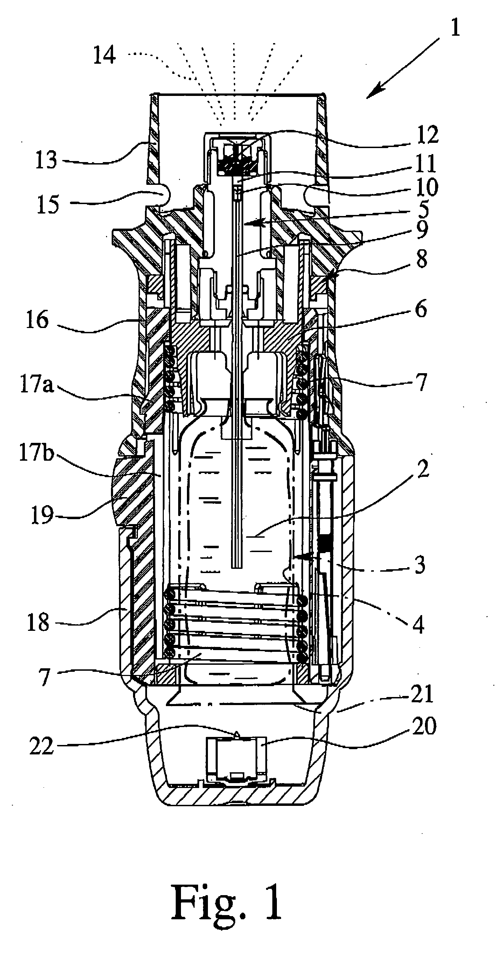

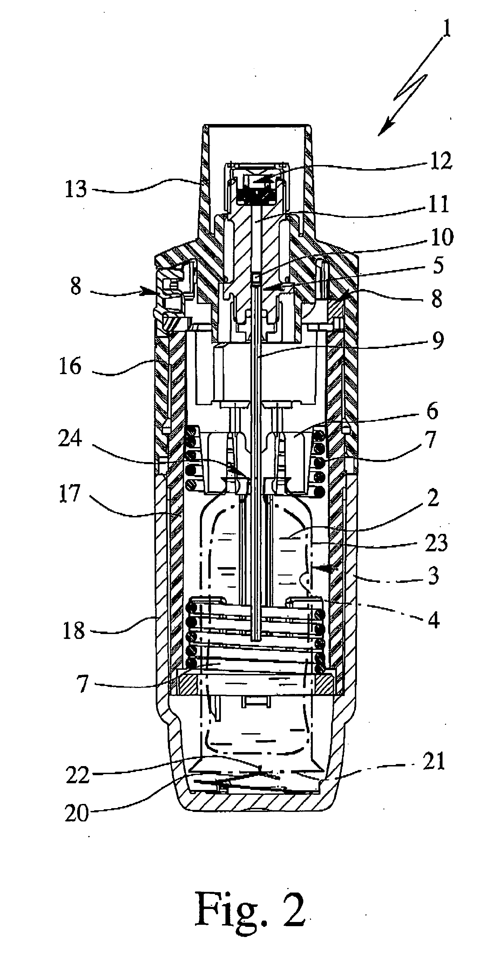

Fig. 1 und 2 zeigen einen vorschlagsgemäßen, tragbaren Inhalator 1 zur treibgasfreien Zerstäubung einer Arzneimittelzubereitung 2 in einer schematischen Darstellung im ungespannten Zustand (Fig. 1) und gespannten Zustand (Fig. 2). Fig. 1 und 2 zeigen den Inhalator 1 mit einem Behälter 3 mit der Arzneimittelzubereitung 2.

Bei Zerstäubung der Arzneimittelzubereitung 2, vorzugsweise einer Flüssigkeit, wird ein Iungengängiges Aerosol 14 (Fig. 1) gebildet, das von einem nicht dargestellten Benutzer bzw, Patienten eingeatmet bzw. inhaliert werden kann. Üblicherweise erfolgt das Inhalieren wenigstens einmal täglich, insbesondere mehrmals täglich, vorzugsweise in vorbestimmten Zeitabständen, insbesondere in Abhängigkeit von der Erkrankung des Patienten,

Der Inhalator 1 weist den vorzugsweise einsetzbaren und ggf. wechselbaren Behälter 3 mit der Arzneimittelzubereitung 2 auf, Der Behälter 3 bildet also ein Reservoir für die zu zerstäubende Arzneimittelzubereitung 2. Vorzugsweise enthält der Behälter 3 eine ausreichende Menge an Arzneimittelzubereitung 2 bzw. Wirkstoff für mehrere Dosen des Arzneimittelzubereitung 2, also mehrere Zerstäubungen oder Anwendungen zu ermöglichen. Ein typischer Behälter 3, wie in der WO 96/06011 A1 offenbart, nimmt ein Volumen von ca. 2 bis 10 ml auf. Hinsichtlich des bevorzugten Aufbaus des Behälters 3 wird ergänzend auf die WO 00/49988 A2 verwiesen.

Der Behälter 3 ist vorzugsweise im wesentlichen zylindrisch bzw. kartuschenartig ausgebildet und von unten, nach Öffnen des Inhalators 1, in diesen einsetzbar und ggf. wechselbar. Er ist vorzugsweise starr ausgebildet, insbesondere wobei das Arzneimittelzubereitung 2 in einem kollabierbaren Beutel 4 im Behälter 3 aufgenommen ist.

Der Inhalator 1 weist ferner eine Fördereinrichtung, insbesondere einen Druckerzeuger 5, zur Förderung und Zerstäubung der Arzneimittelzubereitung 2, insbesondere jeweils in einer vorbestimmten, ggf. einstellbaren Dosiermenge auf,

Der Inhalator 1 bzw. Druckerzeuger 5 weist insbesondere eine Halterung 6 für den Behälter 3, eine zugeördnete, nur teilweise dargestellte Antriebsfeder 7 vorzugsweise mit einem zugeordneten, zur Entsperrung manuell betätigbaren Sperrelement 8, ein Förderelement, vorzugsweise ein als Kapillare ausgebildetes Förderrohr 9, mit einem optionalen Ventil, insbesondere Rückschlagventil 10, eine Druckkammer 11 und/oder eine Austragsdüse 12 insbesondere im Bereich eines Mundstücks 13 auf.

Der Behälter 3 wird über die Halterung 6, insbesondere klemmend oder rastend, so in dem Inhalator 1 fixiert, daß das Förderrohr 9 in den Behälter 3 eintaucht. Die Halterung 6 kann dabei derart ausgebildet sein, daß der Behälter 3 ausgetauscht werden kann.

Beim axialen Spannen der Antriebsfeder 7 wird die Halterung 6 mit dem Behälter 3 und dem Förderrohr 9 bei den Darstellungen nach unten bewegt und die Arzneimittelzubereitung 2 - genauer gesagt die nächste Dosis - aus dem Behälter 3 in die Druckkammer 11 des Druckerzeugers 5 über das Rückschlagventil 10 gesaugt.

Beim anschließenden Entspannen nach Betätigung des Sperrelements 8 wird die Arzneimittelzubereitung 2 in der Druckkammer 11 unter Druck gesetzt, indem das Förderrohr 9 bei nun geschlossenem Rückschlagventil 10 durch Entspannen der Antriebsfeder 7 wieder nach oben bewegt wird und nun als Druckstempel wirkt. Dieser Druck treibt die Arzneimittelzubereitung 2 durch die Austragsdüse 12 aus, wobei es in das vorzugsweise lungengängige Aerosol 14 zerstäubt wird, wie in Fig, 1 und 3 angedeutet.

Der nicht dargestellte Benutzer bzw. Patient kann das Aerosol 14 inhalieren, wobei vorzugsweise Zuluft über mindestens eine Zuluftöffnung 15 in das Mundstück 13 saugbar ist.

Während des Zerstäubungsvorgangs wird der Behälter 3 von der Antriebsfeder 7 wieder in seine Ausgangslage zurückbewegt. Der Behälter 3 führt also eine Hubbewegung während des Spannvorgangs und während des Zerstäubungsvorgangs aus.

Der Inhalator 1 weist insbesondere ein erstes Gehäuseteil (Oberteil) 16 und ein demgegenüber drehbares Innenteil 17 (Fig. 2) mit einem oberen Teil 17a und einem unteren Teil 17b (Fig. 1) auf, wobei an dem Innenteil 17 ein insbesondere manuell betätigbares oder drehbares, zweites Gehäuseteil (Unterteil) 18 vorzugsweise mittels eines Sicherheitsverschlusses bzw. Halteelements 19 lösbar befestigt, insbesondere aufgesteckt, ist. Insbesondere ist der Sicherheitsverschluß bzw. das Halteelement 19 derart ausgebildet, daß ein versehentliches Öffnen des Inhalator 1 bzw, Abziehen des zweiten Gehäuseteils 18 ausgeschlossen ist. Insbesondere muß zum Lösen des zweiten Gehäuseteils 18 das Halteelement 19 gegen Federkraft eingedrückt werden. Zum Einsetzen und/oder Auswechseln des Behälters 3 ist das zweite Gehäuseteil 18 vom Inhalator 1 lösbar. Das zweite Gehäuseteil 18 bildet vorzugsweise ein kappenartiges Gehäuseunterteil und/oder um- bzw. übergreift einen unteren freien Endbereich des Behälters 3.

Das zweite Gehäuseteil 18 kann relativ zum ersten Gehäuseteil 16 gedreht werden, wobei das Innenteil 17 mitgedreht wird. Dadurch wird die Antriebsfeder 7 über ein nicht im einzelnen dargestelltes, auf die Halterung 6 wirkendes Getriebe in axialer Richtung gespannt. Mit dem Spannen wird der Behälter 3 axial nach unten bzw, mit seinem Endbereich (weiter) in das zweite Gehäuseteil 18 bzw. zu dessen stirnseitigem Ende hin bewegt, bis der Behälter 3 eine in Fig. 2 angedeutete Endlage einnimmt. In diesem Zustand ist die Antriebsfeder 7 bzw. der Inhalator 1 gespannt und gesperrt.

Der Inhalator 1 weist vorzugsweise eine Einrichtung zur zwangsweisen Belüftung des Behälters 3 auf.

Beim erstmaligen Spannen erfolgt vorzugsweise ein bodenseitiges Anstechen bzw. Öffnen des Behälters 3. Insbesondere kommt eine axial wirkende, im Gehäuseteil 18 angeordnete Feder 20 am Behälterboden 21 zur Anlage, die mit einem Anstechelement 22 den Behälter 3 bzw. eine bodenseitige, insbesondere gasdichte Versiegelung bei der erstmaligen Anlage zur Belüftung ansticht.

Die Einrichtung zur zwangsweisen Belüftung ist hier also durch das Anstechelement 22 gebildet, das von der Feder 20 gehalten oder gebildet ist. Jedoch sind auch andere konstruktive Lösungen möglich,

Es ist anzumerken, daß bei dem Anstechen zur Belüftung lediglich die Außenhülle des Behälters 3 geöffnet wird. Der Beutel 4 mit der Arzneimittelzubereitung 2 bleibt unbeschädigt. Bei der Entnahme der Arzneimittelformulierung 2 aus dem Beutel 4 über das Förderrohr 9 kollabiert der flexible Beutel 4. Zum Druckausgleich kann die Umgebungsluft über die Belüftungs- bzw. Anstechöffnung in den Behälter 3 nachströmen.

Zur Benutzung des Inhalators 1 muß zunächst der Behälter 3 eingesetzt werden. Dies erfolgt vorzugsweise dadurch, daß das zweite Gehäuseteil 18 entfernt bzw. abgezogen wird. Anschließend wird der Behälter 3 in das Innenteil 17 axial eingeführt bzw. eingeschoben. Hierbei erfolgt ein kopfseitiges Öffnen bzw. Anschließen des Behälters 3. Dies erfolgt durch das Förderelement, also das Förderrohr 9, das eine vorzugsweise vorgesehene, kopfseitige Versiegelung des Behälters 3 durchsticht und anschließend durch ein kopfseitiges Septum des Behälters 3 hindurch in das Innere des Beutels 4 eingerührt wird. So wird die fluidische Verbindung zwischen dem Behälter 3 - genauer gesagt zwischen dem Beutel 4 im Behälter 3 - über das Förderrohr 9 zum Druckerzeuger 5 bzw. zur Druckkammer 11 hergestellt.

Anschließend wird das zweite Gehäuseteil 18 wieder aufgeschoben. Nun kann das erstmalige Spannen des Inhalators 1 erfolgen. Hierbei wird dann der Behälter 3 durch das Anstechelement 22 bodenseitig angestochen, also zwangsweise belüftet, wir bereits erläutert,

Vor der erstmaligen Benutzung erfolgt nach dem Einsetzen bzw. fluidischen Anschließen des Behälters 3 ein vorzugsweise mehrmaliges Spannen und Auslösen des Inhalators 1. Durch dieses sogenannte Primen wird im Förderrohr 9 und im Druckerzeuger 5 bis zur Austragsdüse 12 eventuell vorhandene Luft von der Arzneimittelzubereitung 2 verdrängt. Anschließend ist der Inhalator 1 zur Inhalation bereit.

Die Menge der pro Hub bzw. Zerstäubungsvorgang ausgebrachten Arzneimittelzubereitung 2 beträgt vorzugsweise etwa 10 µl bis 50 µl, insbesondere etwa 10 µl bis 20 µl, ganz besonders bevorzugt etwa 15 µl,

Die Antriebsfeder 7 ist vorzugsweise vorgespannt eingebaut, um einen hohen Förderdruck zu erreichen. Beim vorschlagsgemäßen Inhalator 1 erfolgt das Unterdrucksetzen und Fördern der Arzneimittelzubereitung 2 während des Zerstäubungsvorgangs nämlich vorzugsweise nur durch Federkraft, insbesondere nur durch die Kraft der Antriebsfeder 7.

Der Inhalator 1 ist vorzugsweise derart ausgebildet, daß die Arzneimittelzubereitung 2 im Druckerzeuger 5 bzw. in der Druckkammer 11 bei der Ausgabe einem Druck von 5 MPa bis 60 MPa, insbesondere etwa 10 MPa bis 50 MPa, erreicht. Besonders bevorzugt wird bei der Ausgabe bzw. Zerstäubung der Arzneimittelzubereitung 2 ein Druck von etwa 5 MPa bis 60 MPa, insbesondere etwa 10 bis 30 MPa, an der Austragsdüse 12 bzw. an deren Düsenöffnungen erreicht. Die Arzneimittelzubereitung 2 wird dann in das Aerosol 14 überführt, dessen Tröpfchen einen aerodynamischen Durchmesser von bis zu 20 µm, bevorzugt etwa 3 µm bis 10 µm, aufweisen. Die Zerstäubungswirkung bzw. der Zerstäubungseffekt wird durch sich vorzugsweise kreuzende Strahlen, die von der Austragsdüse 12 abgegeben werden, bewirkt bzw, weiter unterstützt.

Der Inhalator 1 ist vorzugsweise derart ausgebildet., daß das Aerosol 14 mit geringer Geschwindigkeit, insbesondere mit einer Geschwindigkeit von weniger als 2 m/s, besonders bevorzugt von etwa 1,6 m/s oder weniger, ausgegeben wird (jeweils gemessen in 10 cm Abstand von der Austragsdüse 12). Der Inhalator 1 ist also vorzugsweise als SMI ausgebildet, Die geringe Ausgabegeschwindigkeit kann insbesondere durch sich kreuzende Strahlen der Arzneimittelzubereitung 2, die von der Austragsdüse 12 abgegeben werden, und/oder entsprechende Wahl der Federkraft bewirkt bzw. unterstützt werden.

Besonders bevorzugt ist der Inhalator 1 derart ausgebildet, daß die Aerosolerzeugung jeweils über mindestens 1 s, insbesondere über mindestens 1,5 s, dauert. Die Zeitdauer zur Zerstäubung einer Dosis bzw. bei einer Betätigung des Inhalators 1 beträgt also mindestens 1 s, insbesondere über 1,5 s.

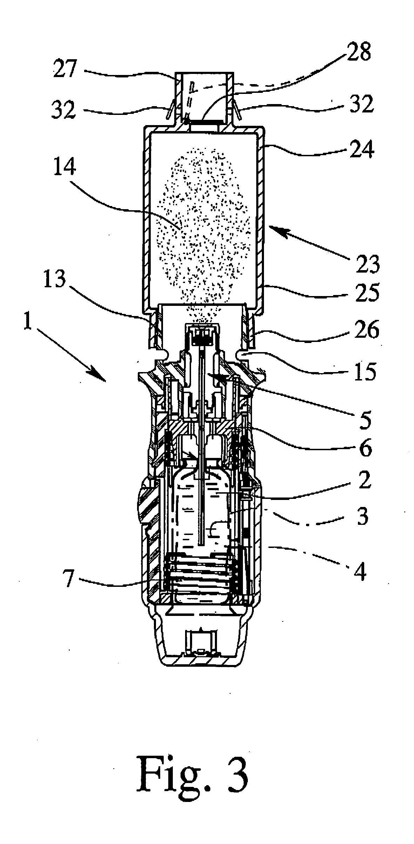

Der Inhalator 1 weist eine Zusatzeinrichtung 23 mit einer Kammer 24 zur Zwischenspeicherung des von dem Inhalator 1 erzeugten Aerosols 14 auf, wie in einem schematischen Schnitt gemäß Fig. 3 gezeigt.

Die Kammer 24 ist stromab der Austragsdüse 12 angeordnet bzw. anordenbar. Sie dient der Aufnahme und Zwischenspeicherung des vom Inhalator 1 erzeugten Aerosols 14.

Vorzugsweise ist die Zusatzeinrichtung 23 bzw. deren Kammer 24 zumindest im wesentlichen zylindrisch, länglich oder konisch ausgebildet.

Vorzugsweise ist die Kammer 24 gegenüber dem Mundstück 13 des Inhalators 1 im Querschnitt vergrößert und/oder erweitert sich zumindest abschnittsweise zum Abgabeende oder freien Ende der Zusatzeinrichtung 23 hin. hierdurch kann erreicht werden, daß das Aerosol 14 in möglichst geringem Umfang auf eine Wandung der Kammer 24 trifft. So kann das Niederschlagen bzw, Abscheiden der zerstäubten Arzneimittelzubereitung 2 an der Kammerwand minimiert werden.

Die Kammer 24 weist vorzugsweise ein Volumen von mehr als 0,1 insbesondere mehr als 0,21, besonders bevorzugt von etwa 0,2 bis 0,61, auf Insbesondere ist die Zusatzeinrichtung 23 bzw. die Größe der Kammer 24 derart an den Inhalator 1 angepaßt, daß das bei einer Betätigung des Inhalators 1 erzeugte Aerosol 14 zumindest im wesentlichen vollständig von der Kammer 24 aufgenommen werden kann, ohne daß sich das Aerosol 14 bzw. die zerstäubte Arzneimittelzubereitung 2 wesentlich an der Kammerinnenwand niederschlägt oder abschneidet.

Die Zusatzeinrichtung 23 weist beim Darstellungsbeispiel vorzugsweise ein Gehäuse 25 auf, das insbesondere länglich und/oder zylindrisch ausgebildet ist.

Die Zusatzeinrichtung 23 bzw. deren Gehäuse 25 ist vorzugsweise zumindest im wesentlichen starr ausgebildet. Jedoch kann die Zusatzeinrichtung 23, die Kammer 24 oder das Gehäuse 25 grundsätzlich auch flexibel, aufblasbar und/oder teleskopierbar ausgebildet sein, insbesondere um den Raumbedarf bei Nichtbenutzung und/oder zum Transport minimieren zu können.

Die Zusatzeinrichtung 23 bzw. das Gehäuse 25 weist vorzugsweise ein Anschlußstück 26 zur Verbindung mit dem Inhalator 1, insbesondere dessen Mundstück 13, auf.

Vorzugsweise ist die Zusatzeinrichtung 23 bzw. das Anschlußstück 26 auf das Mundstück 13 insbesondere klemmend aufsteckbar und/oder von dem Inhalator 1 bzw. Mundstück 13 wieder lösbar. Jedoch kann die Zusatzeinrichtung 23 bedarfsweise auch fest bzw. unlösbar mit dem Inhalator 1 verbunden oder verbindbar sein.

Beim Darstellungsbeispiel weist das Mundstück 13 vorzugsweise mindestens eine Zuluftöfnung 15 auf. Besonders bevorzugt bleibt mindestens eine Zuluf töffnung 15 bei angeschlossener, insbesondere aufgesteckter Zusatzeinrichtung 23 offen, wie in Fig. 3 dargestellt. Dies kann beispielsweise durch entsprechende konische Ausbildung des Mundstücks 13 und dazu komplementärer Ausbildung des Anschlußstücks 26, durch einen nicht dargestellten Anschlag und/oder durch sonstige konstruktive Maßnahmen erreicht werden.

Vorzugsweise ist die Zusatzeinrichtung 23 nicht relativ zum Inhalator 1 verdrehbar. Dies wird beim Darstellungsbeispiel dadurch erreicht, daß das Mundstück 13 des Inhalators 1 eine nicht runde, sondern hier vorzugsweise ovale Außenkontn aufweist, an die das Anschlußstück 26 entsprechend angepaßt ist. Jedoch sind hier auch andere konstruktive Lösungen möglich.

Die Zusatzeinrichtung 23 bzw. das Gehäuse 25 weist vorzugsweise ein Abgabestück 27 zur Abgabe des Aerosols 14 auf Das Abgabestück 27 ist vorzugsweise an dem dem Anschlußstück 26 gegenüberliegenden Ende der Kammer 24 bzw. des Gehäuses 25 angeordnet.

Besonders bevorzugt sind das Anschlußstück 26 und das Abgabestück 27 einstückig an das Gehäuse 25 angeformt. Jedoch sind auch andere konstruktive Lösungen möglich.

Vorzugsweise ist die Kammer 24 bzw. das Gehäuse 25 zumindest teilweise oder insgesamt transparent ausgebildet. Dies ist insbesondere einer Reinigung zuträglich,

Die Zusatzeinrichtung 23 weist vorzugsweise abgabeseitig ein Ventil 28 zum Verhindern eines Rückströmens von Luft in die Kammer 24 auf. So kann verhindert werden, daß beim Ausatmen Luft in die Kammer 24 einströmt, die das Aerosol 14 beispielsweise über das angeschlossene Mundstück 13 und die Zuluftöffnungen 15 nach außen verdrängt.

Bei dem Ventil 28 handelt es sich vorzugsweise um ein Rückschlagventil,

Besonders bevorzugt ist das Ventil 28 in das Anschlußstück 27 eingebaut. Besonders bevorzugt ist das Ventil 28 von der Zusatzeinrichtung 23 bzw. dem Gehäuse 25 lösbar, beispielsweise für Reinigungszwecke.

Alternativ oder zusätzlich zu dem Ventil 28 kann der vorschlagsgemäße Inhalator 1 auch eine (nicht dargestellte) Ventileinrichtung im Bereich des Mundstücks 13 und/oder der Zuluftöffnung(en) 15 aufweisen, um ein Rückströmen von Luft aus der Kammer 24 über das Mundstück 13 und durch die Zuluftöff nung(en) 15 nach außen zu blockieren.

Alternativ oder zusätzlich kann die Zusatzeinrichtung 23 auch eine zusätzliche (nicht dargestellte) Ventileinrichtung, beispielsweise zwischen dem Anschlußstück 26 und dem Mundstück 13 aufweisen, um ein Einströmen von Luft in die Kammer 24 zu gestatten, jedoch ein Ausströmen zu blockieren. In diesem Fall können die Zuluftöffnungen 15 auch ganz entfallen und/oder von der Zusatzeinrichtung 24 abgedeckt bzw, verschlossen werden.

Vorzugsweise weist die Zusatzeinrichtung 23 alternativ oder zusätzlich mindestens ein Ventil 32 zum Ausblasen von Ausatemluft auf, wie in Fig. 3 schematisch dargestellt. Insbesondere zeigt Fig. 3 zwei derartige Ventile 32 im geöffneten Zustand. Ventillappen sind hier von zugeordneten Auslaßöffnungen abgehoben, Das mindestens eine Ventil 32 ist vorzugsweise abgabeseitig, insbesondere am Abgabestück 27 angeordnet. Jedoch sind auch andere konstruktive Lösungen möglich.

Beim Einatmen eines nicht dargestellten Benutzers öffnet das Ventil28, wie gestrichelt in Fig. 3 dargestellt. Die Ventile 32 sind geschlossen. Das Aerosol 14 wird aus der Kammer 24 gesaugt und über das Abgabestück 27 ausgegeben. Beim Ausatmen schließt das Ventil 28 bzw, ist geschlossen. Die Ventile 32 öffnen und gestatten ein Ausblasen der Ausatemluft, ohne daß die Ausatemluft in die Kammer 24 strömt bzw. das Aerosol 14 beeinflußt.

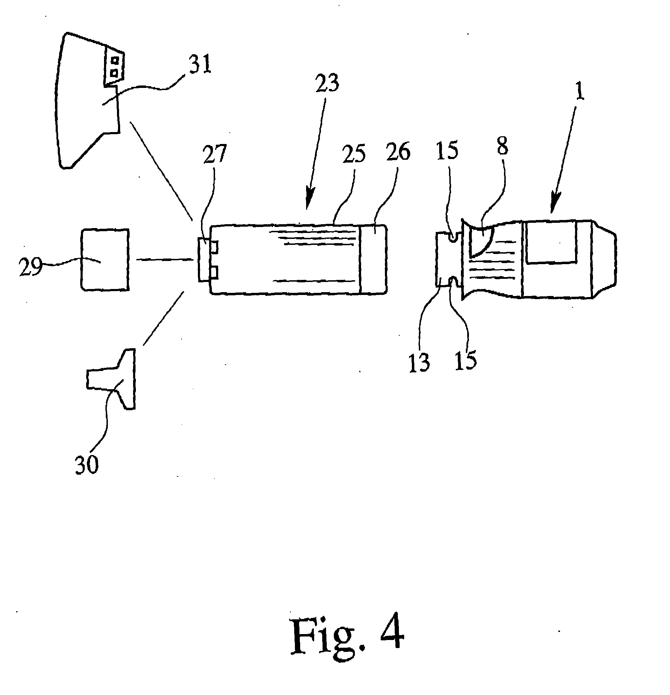

Die Zusatzeinrichtung 23 bzw. deren Abgabestück 27 kann abgabeseitig vorzugsweise mit einem Zusatzmundstück 29, einem Tubus 30 und/oder einer Gesichtsmaske 31 ausgerüstet sein oder werden, wie beispielhaft in Fig. 4 gezeigt. Insbesondere sind verschiedene Endstücke, wie das Zusatzmundstück 29, der Tubus 30 und/oder die Gesichtsmaske 31 wahlweise an die Zusatzeinrichtung 23 bzw. deren Abgabestück 27 anschließbar, besonders bevorzugt aufsteckbar.

Untersuchungen haben gezeigt, daß die vorschlagsgemäße Verwendung der Zusatzeinrichtung 23 beim Inhalator 1, also die vorschlagsgemäße Zwischenspeicherung des vom Inhalator 1 erzeugten Aerosols 14 in einer ausreichend großen Kammer 24 wesentlich dazu beitragen kann, daß ein höherer Anteil des Wirkstoffs beim Inhalieren auch bei Koordinationsproblemen in die Lunge aufgenommen wird.

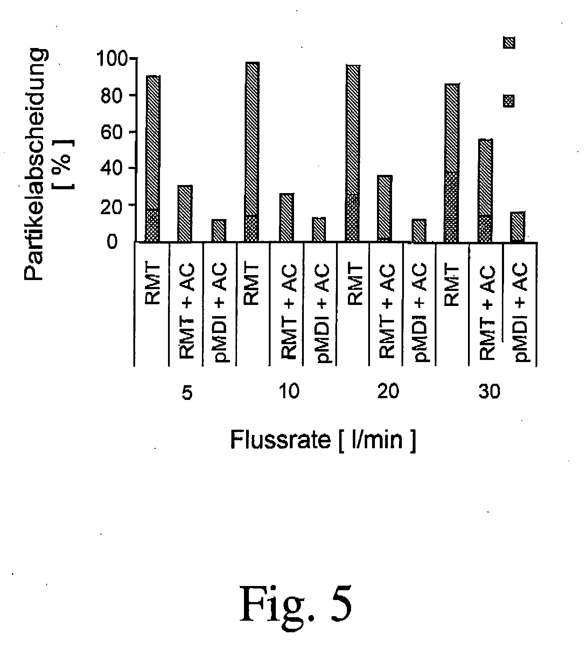

Die beigefügte Fig. 5 veranschaulicht den Wirkstoff anteil, der insgesamt für die Lunge bereitgestellt wird (DeT) und den im Rachen deponierten Wirkstoffanteil (Throat) in Abhängigkeit von der Gesamtdosismenge für verschiedene Inhalatoren.

Die Vertikalachse zeigt den prozentualen Anteil der jeweiligen Dosis, der tatsächlich abgegeben wird, wobei DeT den Anteil darstellt, der beim Inhalieren für die Lunge bereitgestellt wird, und Throat den Anteil datstellt, der im Rachen deponiert wird. Die Wirkstoffdeposition wurde bezogen auf den deklarierten Gehalt unter Verwendung eines Rachenmodells nach Finlay bestimmt. Zugrundegelegt wurde ein inhaliertes Volumen von insgesamt 0,51.

Die Versuche wurden für verschiedene Geräte bzw, Gerätekombinationen und bei verschiedenen Flußraten durchgeführt, wie auf der Horizontalachse aufgetragen. RMT zeigt das Ergebnis des Inhalators 1 ohne Zusatzeinrichtung 23. RMT+AC zeigt das Ergebnis bei Kombination des Inhalators 1 mit der Zusatzeinrichtung 23. pMDI+AC zeigt das Ergebnis bei Kombination eines üblichen MDI mit der Zusatzeinrichtung 23, Die Zahlen 5, 10, 20 und 30 geben geben jeweils die Flußrate in 1/min an.

Aus der Fig. 5 ergibt sich, daß die Verwendung der Zusatzeinrichtung 23 zu einer Verringerung der Rachendeposition führt. Dies ist für pediatrische Anwendungen günstig, da in der Regel im Rachen deponierter Wirkstoff nicht zur Therapie beiträgt, sondern oft zu (systemischen) Nebenwirkungen führt. Der Einsatz der Zusatzeinrichtung 23 ist gut, denn erst oberhalb einer Flußrate von 20 1/min ist bei Verwendung der Zusatzeinrichtung 23 eine geringe Rachendeposition nachweisbar. Bei allen Flußraten ist die Rachendeposition bei der vorschlagsgemäßen Kombination des Inhalators 1 (SMI) mit der Zusatz-einrichtung 23 geringer als die des Inhalators 1 (SMI) alleine. Mit der möglichen therapeutischen Wirkung ist die DeT korrelierbar. Die vorschlagsgemäße Kombination des Inhalators 1 (SMI) mit der Zusatzeinrichtung 23 weist immer eine höhere DeT auf als ein gewöhnlicher MAI mit der Zusatzeinrichtung 23. Der Verlust an DeT durch Einsatz der Zusatzeinrichtung 23 im Vergleich zu dem vorschlagsgemäßen Inhalator 1 ohne Zusatzeinrichtung 23 ist nachweisbar, aber als wesentlich geringer als bei einem gewöhnlichen MDI einzuschätzen. Es wird daher von einer höheren Wirksamkeit bzw. einem höheren Wirkstoff anteil, der die Lunge erreicht, bei der vorschlagsgemäßen Lösung ausgegangen.

Zur Vervollständigung der Offenbarung der vorliegenden Patentanmeldung und zur bevorzugten Ausbildung des Inhalators 1 wird vorsorglich auf den kompletten Offenbarungsgehalt sowohl der WO 91/14468 A1 als auch der WO 97/12687 A1 verwiesen.

Im Gegensatz zu Standgeräten der dergleichen ist der vorschlagsgemäße Inhalator 1 vorzugsweise transportabel ausgebildet, insbesondere handelt es sich um ein mobiles Handgerät.

Aufgrund seiner zylinderähnlichen Form und einer handlichen Größe von weniger als 9 bis 15 cm in der Länge und 2 bis 4 cm in der Breite kann der Inhalator 1 jederzeit vom Patienten mitgeführt werden, Der Vernebler versprüht ein definiertes Volumen der Arzneimittelzubereitung 2 unter Anwendung hoher Drücke durch kleine Düsen, so daß inhalierbare Aerosole 14 entstehen.

Der vorschlagsgemäße Inhalator 1 arbeitet insbesondere rein mechanisch. Jedoch kann der Inhalator 1 grundsätzlich auch auf jede sonstige Art und Weise arbeiten. Insbesondere ist der Begriff "Fördereinrichtung" bzw. "Druckerzeuger" sehr allgemein zu verstehen, Beispielsweise kann der zur Ausgabe und Zerstäubung erforderliche Druck auch durch Treibgas, eine Pumpe oder auf jede sonstige geeignete Art und Weise erzeugt werden.

Der vorschlagsgemäße Inhalator 1 ist insbesondere zur kurzzeitigen Zerstäubung der Arzneimittelzubereitung 2, beispielsweise für ein bis zwei Atemzüge, ausgebildet. Jedoch kann er auch zur längeren oder kontinuierlichen Zerstäubung ausgebildet bzw. einsetzbar sein.

Nachfolgend werden bevorzugte Verbindungen, Bestandteile und/oder Formulierungen der Arzneimittelzubereitung 2 aufgerührt.

Die unten genannten Verbindungen können allein oder in Kombination zur Anwendung in der erfindungsgemäßen Vorrichtung gelangen. In den unten genannten Verbindungen ist W einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGPR-Hemmern, Dopamin-Agonisten, H1-Antihistaminika, PAF-Antagonisten und PI3-Kinase Inhibitoren. Weiterhin können zwei- oder dreifach Kombinationen von W kombiniert werden und zur Anwendung in der erfindungsgemäßen Vorrichtung gelangen. Beispielhaft genannte Kombinationen von W wären;
- W stellt ein Betamimetika dar, kombiniert mit einem Anticholinergika, Corticosteroide, PDE4-Inhibitore, EGFR-Hemmem oder LTD4-Antagonisten,
- W stellt ein Anticholinergika dar, kombiniert mit einem Betamimetika, Corticosteroiden, PDE4-Inhibitoren, EGFR-Hemmcm oder LTD4-Antagonisten,
- W stellt ein Corticosteroiden dar, kombiniert mit einem PDE4-Inhibitoren, EGFR-Hemmern oder LTD4-Antagonisten
- W stellt ein PDE4-Inhibitoren dar, kombiniert mit einem EGFR-Hemmem oder LTD4-Antagonisten
- W stellt ein EGFR-Hemmem dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salinefamol, Salmeterol, Soterenol, Sulphonterol, Terbutaline, Tiaramide, Tolubuterol, Zinterol, CHF-1035, HO-KU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylainino)-1-hydroxy-ethyl)-8-hydroxy-1H-quinolin-2-on
- 4-Hbydroxy-7-[2-{[2-{[3-(2-phenylethoxy)propyl]sulphonyl}ethyl]-amino}ethyl)-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-ben2oxazin-8-yl]-2-[3-(4-N,N-dimethylaminophenyl)-2-methyl-2-propylamino]ethanol
- 1-[2H-5-hydroxy-3-oxe-4H-1,4-benzoxazin-8-yl]-2-(3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3'(4-methoxyphenyl)-1,2,4-triazol-3-yl]-2-methyl-2-butylamino}ethanol
- 5-Hydroxy-8-(1-hydroxy-2-isopxopylaminobutyl)-2H-1,4-benzoxazin-3-(4H)-on
- 1-(4-Amino-3-chlor-5-trifluormethylphenyl)-2-tert-butylamino)ethanol
- 6-Hydroxy-8-{1-hydroxy-2-[2-(4-methoxy-phenyl)-1,1-dimethyl-ethylamino)-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxyethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylaminol-ethyl}-4H-benzo[1,4]oxazin-3-on
- 8-{2-[2-(4-Ethyl-phenyl)-1,1-dimethyl-ethylamino)-1-hydroxy-ethyl}-6-hydroxy-4H-benzo[1,4]oxazin-3-on
- 8-{2-[2-(4-Ethoxy-phenyl)1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-3-fluorophenyl)-2-(tert,-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxyethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-bydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfmdungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalien, bevorzugt das Bromidsalz, Flutropiumsalzen, bevorzugt das Bromidsalz, Ipratropiumsalzen, bevorzugt das Bromidsalz, Cxlycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel AC-1 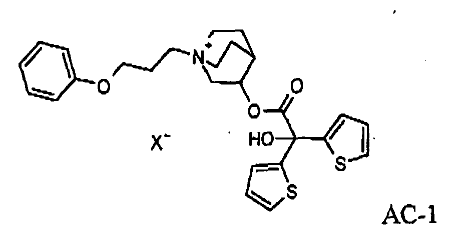 worin X - ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel AC-1-en 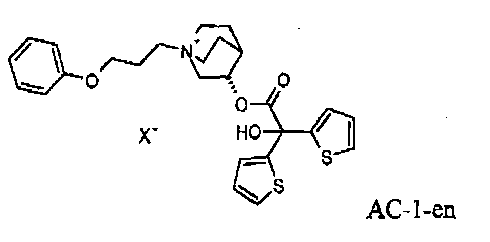 enthalten, worin X - die vorstehend genannten Bedeutungen aufweisen kann. Weiterhin bevorzugte Anticholinergika sind ausgewählt aus den Salzen der Formel AC-2 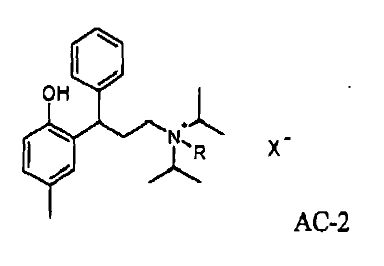 worin R entweder Methyl oder Ethyl bedeuten und worin X - die vorstehend genannte Bedeutungen aufweisen kann. In einer alternativen Ausführungform kann die Verbindung der Formel AC-2 auch in Form der freien Base AC-2-base vorliegen.

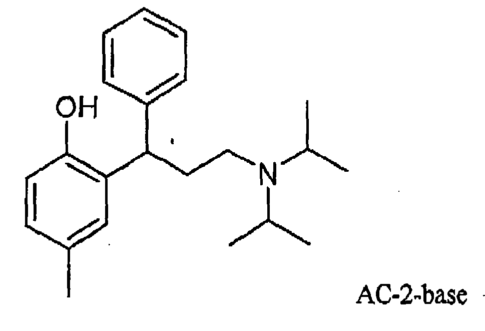

Weiterhin genannte Verbindungen sind;
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'- Difluorbenzilsäurescopinester- Methobromid
- 3,3'-Difluorbenzilsäuretropenolester-Methobromid
- 3,3 '- Difluorbenzilsäurescopinester- Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbensäurescopinester-Methobromid
- 9-Fluor-fluoxen-9-carbonsäurescopinester-Methobromid
- 9-Methyl-fluoren-9-carbonsäuretropenolester-Metlxobromid
- 9-Methyl-fluoren-9-carbonsäurescopinester-Methobromid
- Benzilsäurecyclopropyltropinester-Methobromid
- 2,2-DiphenyIpropionsäurecyclopropyItropmester-Methobromid
- 9-Hydroxy-xanthen-9-carbonsäureeyclopropyltropinester-Methobromid
- 9-Methyl-fluoren-9-earbonsäurecyclopxopyltropinester-Methobromid
- 9-Methyl-xänthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-earbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-earbonsäuretopenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-füran-3 S-yl)ester,
- 6α,9α-difluoro-11β-hydroxy-16α-methyl-3-oxo-17α-(2,2,3,3-tertamethylcyclopropylcarbonyl)oxy-androsta- 1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate, Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, C1-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8'-methoxy-2-methylbenzo[s][1,6]naphthyridül-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulibnat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2- hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl), propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Henuner gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorpbenyl)amino]-6-{[4-(morpholin-4-yl)-1-oxo-2-buten-1-yl)amino}-7-cyclopropylmethoxy-chinazolin
- 4-[(3-Chlor-4-fluorphenyl)amino)-6-{[4-(N,N-diethylamino)-1-oxo-2-buten-1-yl]amino}-7-cycloproplmethoxy-chinazolin
- 4-[(3-Chlor-4-fluorphenyl)amino]-6-{[4-(N,N-dimethylamino)-1-oxo-2-buten-l-yl]ammo}-7-cyclopropylmethoxy-chmazolm
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl)amino)-7-cyclopropylmethoxy-chinazolin
- 4-[(3-Chlor-4-fluor-phenyl)amino]-6-[2-((S)-6-metllyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)arnino]-6-({4-[N-(2-inethoxy-ethyl)-N-methyl-amino]-l-oxo-2--buten-l-yl}ammo)-7-cyclopropylmethoxy-chinazolm
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino)-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino)-7-((S)-tetrahydrofuran-3-yloxy)-chinazolin
- 4-[(3-Chlor-4-auorphenyl)amino]-6-({4-[N-(2-methoxy-ethyl)-N-methyl-amino]-1-oxo-2-buten-1-yl}amino)-7-cyclopentyloxy-chinazoiln
- 4-[(3-Chior-4-auorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino]-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidm
- 3-Gyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino)-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino)-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy)-7-methoxy-chinazolin
- 4-[(3-Chlor-4-fluor-phenyl)amino]-6-(pipendin-3-yloxy)-7-methoxy-chinazolin
- 4-[(3-Chlor-4-fluor-phenyl)amino]-6-[1-(2-acetylamino-ethyl)-pipendin-4-yloxy]-7-methoxy-chinazolm
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-chlor-4-fluor-phenyl)amino]-6-(terahydropyran-4-ytoxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[{3-Chlor-4-fluor-phenyl)amino]-6-{trans-4-[(morpholin-4-yl)sulfonylamino]-cyclohexan-1-yloxy}-7-methoxy-chinazolin
- 4-[{3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(pipendin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-oyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino)-6-(trans-4-eansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-znethoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholm-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[{3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethy]-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{ 1-[(N-methyl-N-2-methoxyethyl-amino)carbonyl]-pipendin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(2-(2,2-dimethyl-6-oxo-morpholin-4-yl)-ethoxy]-7-[(S)-(tetrahydrofuran-2-yl)methoxy]-chinazolin
- 4-[(3-Chlor-4-fluer-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizirn, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 A1 oder CA 2297174 A1 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

### Bezugszeichenliste

- 1: Inhalator
- 2: Arzneimittelzubereitung
- 3: Behälter
- 4: Beutel
- 5: Druckerzeuger
- 6: Halterung
- 7: Antriebsfeder
- 8: Sperrelement
- 9: Förderrohr
- 10: Rückschlagventil
- 11: Druckkammer
- 12: Austragsdüse
- 13: Mundstück
- 14: Aerosol
- 15: Zuluftöffnung
- 16: erstes Gehäuseteil (Oberteil)
- 17: Innenteil
- 17a: oberer Teil des Innenteils
- 17b: unterer Teil des Innenteils
- 18: zweites Gehäuseteil (Unterteil)
- 19: Halteelement
- 20: Feder (im Gehäuseunterteil)
- 21: Behälterboden
- 22: Anstechelement
- 23: Zusatzeinrichtung
- 24: Kammer
- 25: Gehäuse
- 26: Anschlußstück
- 27: Abgabestück
- 28: Ventil
- 29: Zusatzmundstück
- 30: Tubus
- 31: Gesichtsmaske
- 32: Ventil

## Patentansprüche

1. Tragbarer Inhalator (1) zur treibgasfreien Zerstäubung einer Arzneimittelzubereitung (2), mit einem vorzugsweise als Pumpe ausgebildeten und/oder mechanisch arbeitenden Druckerzeuger (5) und mit einer Austragsdüse (12) zur Ausgabe der zerstäubten Arzneimittelzubereitung (2) als Aerosol (14) insbesondere in ein Mundstück (13),
**dadurch gekennzeichnet,**
**daß** der Inhalator (1) eine Zusatzeinrichtung (23) mit einer Kammer (24) zur Zwischenspeicherung des Aerosols (14) aufweist, wobei die Kammer (24) stromab der Austragsdüse (12) angeordnet oder anordenbar ist,

2. Inhalator nach Anspruch 1, **dadurch gekennzeichnet, daß** die Zusatzeinrichtung (23) auf das Mundstück (13) vorzugsweise klemmend aufsteckbar und/oder von dem Inhalator (1) bzw. Mundstück (13) lösbar ist.

3. Inhalator nach Anspruch 1 oder 2, **dadurch gekennzeichnet, daß** die Zusatzeinrichtung (23) bzw. die Kammer (24) zumindest im wesentlichen zylindrisch, länglich oder konisch ausgebildet ist.

4. Inhalator nach einem der voranstehenden Ansprüche, **dadurch gekennzeichnet, daß** die Kammer (24) ein Volumen von mehr als mindestens 0,1 1, vorzugsweise mehr als 0,21, insbesondere von etwa 0,2 bis 0,6 1, aufweist.

5. Inhalator nach einem der voranstehenden Ansprüche, **dadurch gekennzeichnet, daß** das Mundstück (13) mindestens eine Zuluftöffnung (15) aufweist, die bei angeschlossener, insbesondere aufgesteckter Zusatzeinrichtung (23) offen bleibt.

6. Inhalator nach einem der voranstehenden Ansprüche, **dadurch gekennzeichnet, daß** die Zusatzeinrichtung (23) insbesondere abgabeseitig ein Ventil (28) zum Verhindern eines Rückströmens von Luft in die Kammer (24) und/oder das Mundstück (13) und/oder zum Absaugen des Aerosols und/oder mindestens ein Ventil (32) zum Ausblasen von Ausatemluft aufweist,

7. Inhalator nach einem der voranstehenden Ansprüche, **dadurch gekennzeichnet, daß** die Zusatzeinrichtung (23) abgabeseitig vorzugsweise wahlweise mit einem Zusatzmundstück (29), einem Tubus (30) und/oder einer Gesichtsmaske (31) ausgerüstet oder ausrüstbar ist.

8. Inhalator nach einem der voranstehenden Ansprüche, **dadurch gekennzeichnet, daß** der Inhalator (1) derart ausgebildet ist, daß das Aerosol (14) mit geringer Geschwindigkeit, insbesondere mit einer Geschwindigkeit von weniger als 2 m/s, besonders bevorzugt von etwa 1,6 m/s oder weniger, in einem Abstand von 10 cm von der Austragsdüse (12), ausgegeben wird.

9. Inhalator nach einem der voranstehenden Ansprüche, **dadurch gekennzeichnet, daß** der Inhalator (1) derart ausgebildet ist, daß er pro Betätigung oder als Dosis jeweils 10 bis 50 µl der Arzneimittelzubereitung (2) über eine Zeitdauer von mindestens 1 s, insbesondere über 1,5 s, ausgibt und zerstäubt.

10. Inhalator nach einem der voranstehenden Ansprüche, **dadurch gekennzeichnet, daß** der Inhalator (1) derart ausgebildet ist, daß er definierte Mengen der Arzneimittelzubereitung (2) unter Drücken von 10 bis 60 MPa zerstäubt.

11. Inhalator nach einem der voranstehenden Ansprüche, **dadurch gekennzeichnet, daß** die Druckerzeugung bzw. Zerstäubung durch Federkraft erfolgt.

12. Verwendung einer Zusatzeinrichtung (23) mit einer Kammer (24) bei einem tragbaren Inhalator (1), wobei der Inhalator (1) zur treibgasfreien Zerstäubung einer Arzneimittelzubereitung (2) mittels eines als Pumpe ausgebildeten Druckerzeugers (5) ausgebildet ist, wobei die Arzneimittelzubereitung (2) über eine Austragsdüse (12) als ein Aerosol (14) mit einer Geschwindigkeit von weniger als 2 m/s in einem Abstand von 10 cm von der Austragsdüse (12) ausgebbar ist, wobei die Kammer (24) stromab der Austragsdüse (12) an den Inhalator (1) zur Zwischenspeicherung der zerstäubten Arzneimittelzubereitung (2) angeschlossen wird.
